(19) Europäisches Patentamt
European Patent Office
Office européen des brevets

(11) Publication number: **0 095 262**
**B1**

(12) **EUROPEAN PATENT SPECIFICATION**

(45) Date of publication of patent specification: **03.12.86**

(21) Application number: **83302493.8**

(22) Date of filing: **04.05.83**

(51) Int. Cl.[4]: **C 07 D 451/04,**
C 07 D 451/02,
C 07 D 451/14,
C 07 D 451/00, A 61 K 31/46

(54) **Azabicycloalkane derivatives, their preparation and medicaments containing them.**

(30) Priority: **11.05.82 GB 8213588**
**11.05.82 GB 8213549**

(43) Date of publication of application:
**30.11.83 Bulletin 83/48**

(45) Publication of the grant of the patent:
**03.12.86 Bulletin 86/49**

(84) Designated Contracting States:
**CH DE FR GB IT LI NL**

(56) References cited:
**EP-A-0 013 138**
**EP-A-0 031 219**
**EP-A-0 042 705**
**EP-A-0 055 524**
**EP-A-0 067 615**
**EP-A-0 068 700**
**EP-A-0 069 481**
**EP-A-0 069 482**
**GB-A-2 042 522**
**US-A-2 800 483**

(73) Proprietor: **BEECHAM GROUP PLC**
**Beecham House Great West Road**
**Brentford Middlesex TW8 9BD (GB)**

(72) Inventor: **Markwell, Roger Edward**
**22 Venmore Drive**
**Great Dunmow Essex (GB)**
Inventor: **Hadley, Michael Stewart**
**9 Coney Gree**
**Sawbridgeworth Herts (GB)**
Inventor: **Blaney, Frank Edward**
**19C Clissold Court Green Way Close**
**London, N4 2EZ (GB)**

(74) Representative: **Russell, Brian John et al**
**European Patent Attorney**
**Beecham Pharmaceuticals**
**Great Burgh Yew Tree Bottom Road**
**Epsom Surrey, KT18 5XQ (GB)**

**EP 0 095 262 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel benzamides having useful pharmacological activity, to a process for their preparation, and to pharmaceutical compositions containing them.

U.S. Patent No. 4,273,778 discloses a class of benzamides of formula (A):

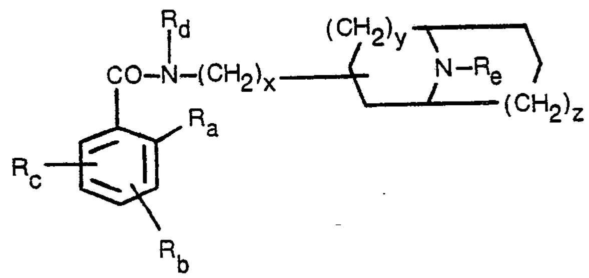

(A)

wherein:

$R_a$ is alkoxy;

$R_b$ and $R_c$ are hydrogen, halogen, $CF_3$, acyl, acylamino or amino, aminocarbonyl or aminosulphone, optionally substituted by alkyl, alkylsulphone or nitro;

$R_d$ is hydrogen or alkyl;

$R_e$ is alkyl or $—(CH_2)_sR_f$ where s is 0 to 2 and $R_f$ is cycloalkyl, or $—(CH_2)_tR_g$ where t is 1 or 2 and $R_g$ is alkenyl or phenyl optionally substituted by one or two substituents selected from alkyl, alkoxy, trifluoromethyl and halogen; and x, y and z are 0 to 2.

Such compounds and their pharmaceutically acceptable salts are described as having dopamine antagonist activity.

British Patent Publication No. 2,042,522 A discloses nortropanes of formula (B):

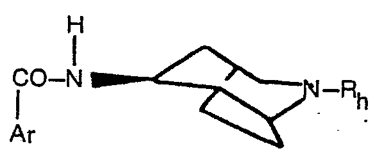

(B)

wherein Ar represents variously substituted phenyl or pyrimidinyl and $R_h$ represents benzyl optionally substituted by alkyl, trifluoromethyl, chloro, bromo, fluoro, thiophenylmethyl or furanylmethyl. Such compounds are described as having neuroleptic properties.

European Patent Publication No. 26749 discloses a class of cyclic benzamides of formula (C):

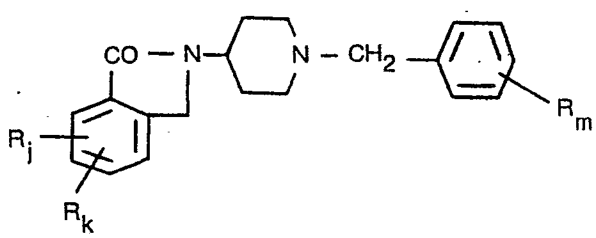

(C)

wherein $R_j$ and $R_k$ are hydrogen, halogen, alkyl, alkoxy or trifluoromethyl and $R_m$ is hydrogen, halogen, alkyl, alkoxy or trifluoromethyl. Such compounds are described as having neuropharmacological activity.

European Patent Publication No. 31219 discloses compounds of formula (A), in which the benzamide moiety is replaced by an anilide moiety of formula (D):

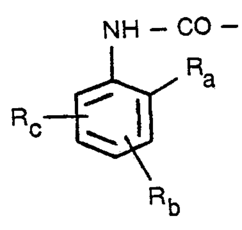

(D)

wherein $R_a$ to $R_c$ are substantially as defined for formula (A). The anilide compounds also have dopamine antagonist activity.

European Patent Publication No. 41817 discloses compounds of formula (A), in which the bicyclic ring system is replaced by a system of formula (E):

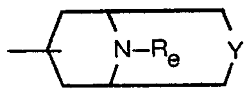

(E)

wherein $R_e$ is substantially as defined for formula (A) and Y is oxygen or sulphur. Such compounds also have dopamine antagonist activity.

European Patent Publication No. 42705 discloses compounds of formula (A), in which $R_c$ is replaced by alkylsulphinyl and which are described as dopamine antagonists.

A novel class of benzamides having a bicyclic side chain have now been discovered. Such compounds contain a substitution on the bridge of the bicyclic ring system and have dopamine antagonist activity.

Accordingly, the present invention provides a compound of formula (I), or a pharmaceutically acceptable salt thereof, or a solvent adduct or N-oxide thereof, or a solvate of any of the foregoing:

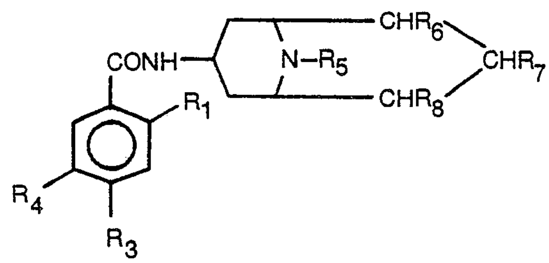

(I)

wherein:

$R_1$ is $C_{1-6}$ alkoxy;

$R_3$ is carboxylic $C_{1-7}$ acylamino or amino;

$R_4$ is halogen or hydrogen;

$R_5$ is —$CH_2R_{11}$, $R_{11}$ being phenyl substituted by one substituent selected from halogen and $C_{1-4}$ alkyl; and

one of $R_6$, $R_7$ and $R_8$ alkyl and the other two are the same or different and are hydrogen or $C_{1-4}$ alkyl.

Preferred examples of $R_1$ include methoxy, ethoxy, *n*- and *iso*-propoxy. Most preferably $R_1$ is methoxy.

$R_3$ is preferably $C_{1-4}$ alkanoylamino such as formylamino, acetylamino, propionylamino, *n*- or *iso*-butyrylamino, or amino. $R_4$ is preferably hydrogen, chloro or bromo. Most preferably, $R_4$ is hydrogen, chloro or bromo, and $R_3$ is amino.

Particularly preferred moieties falling within formula (I) include those wherein $R_1$ is methoxy, $R_3$ is amino, and $R_4$ is chloro or hydrogen.

Additionally, particularly preferred moieties falling within formula (I) are those in which $R_1$ is methoxy, $R_3$ is amino optionally N-substituted by $C_{1-4}$ alkanoyl, and $R_4$ is chloro.

When $R_{11}$ phenyl in $R_5$ is substituted by $C_{1-4}$ alkyl, the preferred examples of $C_{1-4}$ alkyl include methyl, ethyl, *n*- and *iso*-propyl, and *n*-, *iso*-, *sec*- and *tert*-butyl; methyl however is most preferred.

The most preferred examples of $R_5$ are those wherein $R_{11}$ is mono-p-substituted phenyl. Examples of preferred p- substituents include methyl, fluoro, chloro and bromo.

*p*-Fluorobenzyl, *p*-chlorobenzyl and *p*-methylbenzyl are especially preferred examples of $R_5$.

Preferably each of $R_6$, $R_7$ and $R_8$ are in the exo-position.

Preferred examples for one of $R_6$, $R_7$ and $R_8$ are methyl, ethyl and *n*-propyl.

Preferred examples for the other two of $R_6$, $R_7$ and $R_8$ are hydrogen, methyl, ethyl and *n*- and *iso*-propyl.

Within formula (I) is a sub-class of compounds of formula (II), or a pharmaceutically acceptable salt thereof, or a solvate of either of the foregoing:

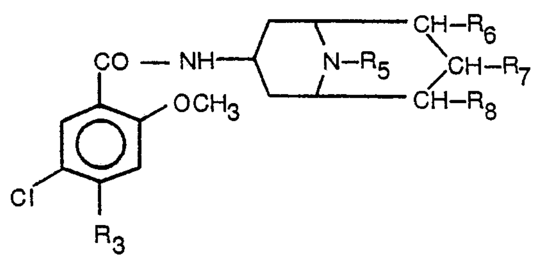

(II)

wherein $R_3$ is amino or $C_{1-4}$ alkanoylamino, and $R_5$ to $R_8$ are as defined hereinbefore.

It is especially preferred that the $R_{11}$ phenyl ring is mono-substituted in the para-position and/or that the substituent is chloro, fluoro or methyl.

Within formula (II) and sub-classes thereof, there are further sub-classes of compounds, wherein one of $R_6$, $R_7$ and $R_8$ is methyl or ethyl and the other two are the same or different and are methyl, ethyl or hydrogen.

The compounds of formulae (I) and (II) have chiral or prochiral centres and thus are capable of existing in a number of stereoisomeric forms. All such isomers and mixtures thereof are included within the present invention.

It is preferred that —NH— is in the equatorial orientation to the bicyclic ring system as shown. Consequently, the preferred configuration for all compounds is of formula (III) or (IV).

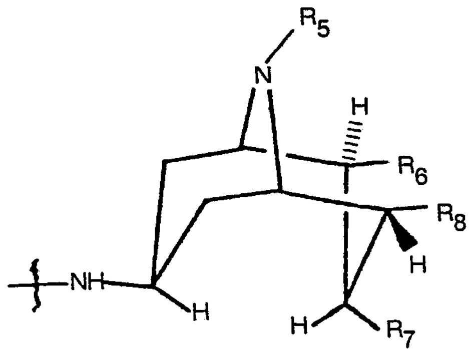

Chair—Chair (III)

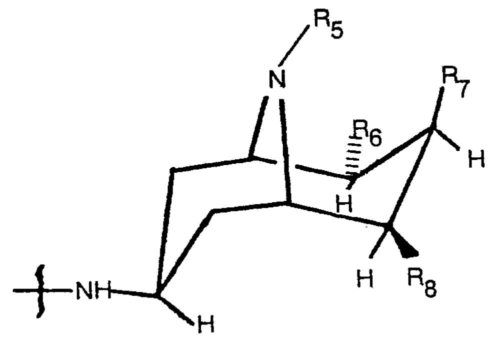

Chair—Boat (IV)

wherein $R_5$ to $R_8$ are as defined hereinbefore and wherein each of $R_6$, $R_7$ and $R_8$ in both formulae (III) and (IV) are shown in the exo-position, and their enantiomers.

The pharmaceutically acceptable salts of the compounds of this invention include acid addition salts with conventional acids such as hydrochloric, hydrobromic, phosphoric, sulphuric, citric, tartaric, lactic and acetic acid, and quaternary ammonium salts with alkyl, phenalkyl and cycloalkyl halides. Suitable examples of such quaternising agents include methyl, ethyl, *n*- and *iso*-propyl, benzyl, phenethyl, chlorides, bromides and iodides. Solvent adducts include hydrates and adducts with organic solvents such as chloroform.

The present invention also provides a process for preparing a compound of formula (I), or a pharmaceutically acceptable salt or N-oxide thereof, which comprises reacting a compound of formula (V):

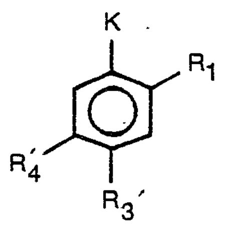

(V)

with a compound of formula (VI):

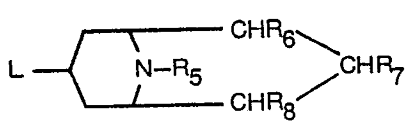

(VI)

wherein $R_5$ to $R_8$ are as defined;

$R_1$ is as defined; K is $COQ_1$, in which $Q_1$ is a leaving group, and L is amino;

$R_3'$ and $R_4'$ are the same or different and are selected from hydrogen, halogen, carboxylic $C_{1-7}$ acylamino, nitro, hydroxy or amino; converting $R_3'$ and/or $R_4'$ as necessary to $R_3$ and $R_4$ respectively; optionally converting $R_3$ or $R_4$ in the thus formed compound to another $R_3$ or $R_4$ within formula (I); and optionally forming a pharmaceutically acceptable salt or N-oxide of the thus formed compound.

The leaving group $Q_1$ is a group that is readily displaceable by a nucleophile. Examples of such groups are hydroxy and halogen such as chloro and bromo.

If a leaving group is hydroxy, then the reaction is preferably carried out in an inert non-hydroxylic solvent, such as benzene, toluene or diethyl ether in the presence of a dehydrating catalyst, such as a carbodiimide, for example dicyclohexylcarbodiimide. The reaction may be carried out at a non-extreme temperature such as −10 to 100°C, for example 0 to 80°C.

If a leaving group is a halide, then the reaction is preferably carried out at a non-extreme temperature in an inert non-hydroxylic solvent, such as benzene, toluene or diethyl ether. It is also preferably carried out

in the presence of an acid acceptor, such as an organic base, in particular a tertiary amine, such as triethylamine, trimethylamine, pyridine or picoline, some of which can also function as the solvent. Alternatively, the acid acceptor can be inorganic, such as calcium carbonate, sodium carbonate or potassium carbonate.

Preferably $Q_1$ is halogen, such as chloro.

The compounds of formula (V) are either known compounds or can be prepared analogously to the preparation of structurally similar known compounds, by known methods.

Compounds of formula (VI) can be prepared analogously to the preparation of structurally similar known compounds. Such compounds can be prepared by reduction of the oxime of formula (VII):

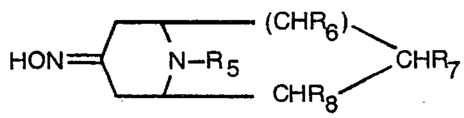

(VII)

wherein $R_5$ to $R_8$ are as hereinbefore defined.

The reducing agent may be sodium and amyl alcohol, lithium aluminium hydride or a catalytic reducing agent, such as hydrogen and nickel.

The oxime of formula (VII) can be prepared by reacting the compound of formula (VIII):

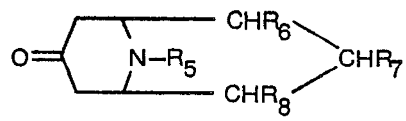

(VIII)

wherein $R_5$ to $R_8$ are as defined hereinbefore, with hydroxylamine.

Compounds of formula (VIII) wherein $R_5$ to $R_8$ are as hereinbefore defined, can be prepared by reacting a compound of formula (IX):

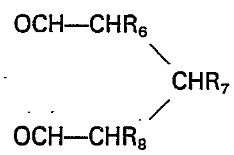

(IX)

wherein $R_6$ to $R_8$ are as defined, with a compound of formula (X):

$$H_2N—R_5 \qquad (X)$$

wherein $R_5$ is as hereinbefore defined, and acetone-1,3-dicarboxylic acid.

The reaction is an example of the Robinson-Schopf reaction and is preferably carried out at a pH of 5 in a solution of sodium acetate buffer in water.

Compounds of formula (X) and 1,3-acetone dicarboxylic acid are known compounds.

Compounds of formula (IX), the dialdehydes, are known compounds or are derivable therefrom or can be prepared analagously to the preparation of structurally similar known compounds.

Compounds of formula (IX), wherein $R_6$, $R_7$ and $R_8$ are as hereinbefore defined, can be prepared by acid hydrolysis of a compound of formula (XI):

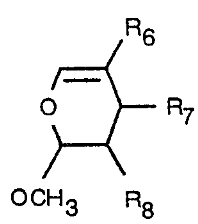

(XI)

wherein $R_6$ to $R_8$ are as hereinbefore defined, in accordance with the procedure described by K. Alder *et al* in *Annal., 620,* 73 (1959).

Compounds of formula (XI) are known compounds or are derivable therefrom or can be prepared analogously to the preparation of known, structurally-similar compounds.

Compounds of formula (I), wherein $R_3'$ or $R_4'$ is convertible to $R_3$ or $R_4$ within formula (I), and where $R_3$ or $R_4$ is convertible to other $R_3$ or $R_4$, are useful intermediates. A number of such conversions is possible not only for the end compounds of formula (I), but also for their intermediates as follows:

(a) a hydrogen substituent is convertible to a nitro substituent by nitration;

(b) a nitro substituent is convertible to an amino substituent by reduction;
(c) a $C_{1-7}$ acylamino substituent is convertible to an amino substituent by deacylation;
(d) an amino substituent is convertible to a $C_{1-4}$ acylamino substituent by acylation;
(e) a hydrogen or hydroxy substituent is convertible to a halogen substituent by halogenation; and
(f) a fluoro or chloro substituent is convertible to an amino substituent by reaction with ammonia.

Conversions (a) to (f) are only exemplary and are not exhaustive of the possibilities.

In regard to (a), nitration is carried out in accordance with known procedures.

In regard to (b), the reduction is carried out with a reagent suitable for reducing nitroanisole to aminoanisole. The reduction is a convenient method for the preparation of the correspondingly substituted compounds of formula (I), wherein X is NH and Y is CO. However, when X is CO and Y is NH, it is more convenient to use deacylation (c).

In regard to (c), deacylation is carried out by treatment with a base, such as an alkali metal hydroxide.

In regard to (d), the acylation is carried out with an acylating agent, such as the corresponding acid or acid chloride. Formylation is carried out with the free acid.

In regard to (e), halogenation is carried out with conventional halogenating agents for aromatic hydrogen or hydroxy substituents.

In regard to (f), the amination is carried out under conventional conditions using an inert solvent such as $CH_2Cl_2$ or an excess of ammonia also functioning as the solvent.

Before carrying out any of these conversions, the effect, if any, on other substituents should be considered and such reagents as are appropriate should be selected together with the adoption of such precautionary measures as are necessary.

Compounds of formula (I), wherein $R_5$ is benzyl substituted in the phenyl ring as previously defined are convertible one into another, and convertible from corresponding compounds where other, optionally substituted, benzyl is present in place of $R_5$, via a secondary amine.

Accordingly, the present invention further provides a process for preparing a compound of formula (I), or pharmaceutically acceptable salt thereof, or a solvent adduct or N-oxide thereof, which comprises reacting a secondary amine of formula (XII):

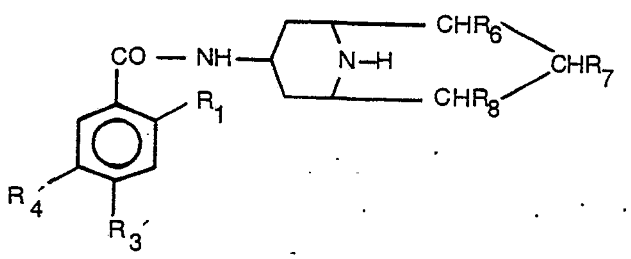

(XII)

wherein $R_1$, $R_3'$ and $R_4'$, and $R_6$ to $R_8$ are as hereinbefore defined, with a compound $PR_5$, wherein $R_5$ is benzyl substituted in the phenyl ring by one substituent selected from halogen and $C_{1-4}$ alkyl, and P is a leaving group; converting $R_3'$ and/or $R_4'$ as necessary to $R_3$ and $R_4$ respectively, optionally converting $R_3$ and $R_4$ in the thus formed compound to another $R_3$ or $R_4$ within formula (I); and optionally forming a pharmaceutically acceptable salt, solvent adduct or N-oxide of the thus formed compound.

The leaving group P is a group that is readily displaceable by a nucleophile. Preferred examples of P are chloro, bromo, iodo, mesyl and tosyl. Most preferred examples are bromo and chloro.

The reaction is normally carried out under conventional N-aralkylation conditions, for exampole in an inert solvent, such as dimethylformamide, in the presence of an acid acceptor, such as potassium carbonate. Generally, the reaction is carried out at a non-extreme temperature, such as ambient temperature or slightly above.

The compound of formula (XII), as defined hereinbefore, is prepared by conventional transition metal catalysed hydrogenolysis of a compound of formula (XIII):

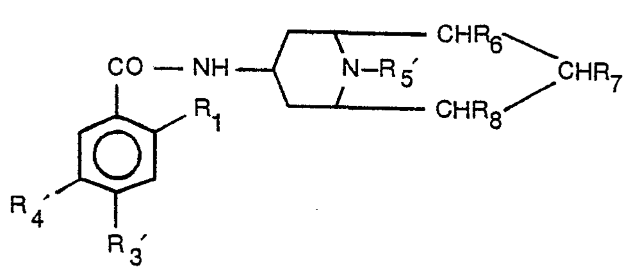

(XIII)

wherein $R_5'$ is benzyl optionally substituted in the phenyl ring by one or two substituents selected from $C_{1-4}$ alkoxy, trifluoromethyl, halogen, nitro, carboxy, esterified carboxy and $C_{1-4}$ alkyl optionally substituted by hydroxy, $C_{1-4}$ alkoxy, carboxy, esterified carboxy or *in vivo* hydrolysable acyloxy, and $R_1$, $R_3'$ and $R_4'$, and

$R_6$ to $R_8$ are as previously defined. Such hydrogenolysis is preferably not carried out on a compound of formula (XIII), wherein either of $R_3'$ and $R_4'$ is halogen.

Instead of interconverting the substituted benzyl substituent of $R_5$ in a compound of formula (I), it is preferred that such interconversion is carried out on the compound of formula (VI). It is desirable to protect the primary amine function prior to interconversion. A suitable protecting group is $C_{2-7}$ alkanoyl, which is readily removable after interconversion by acid hydrolysis.

As mentioned hereinbefore the compounds of formula (I) exist in a number of stereoisomeric forms, in particular the moiety —NH— can be in the axial or the equatorial orientation to the bicyclic ring system resulting in α- and β-isomers respectively. A mixture of such isomers can be obtained by a non-stereospecific process and then the desired isomer separated conventionally from the mixture by, for example, chromatography. Alternatively, the α- and β-isomers can be prepared separately from the α- or β-isomer of formula (VI).

By way of example, the α-isomer of a compound of formula (XIV):

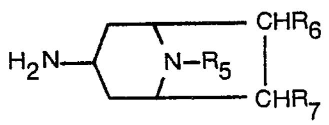

(XIV)

can be prepared by catalytic hydrogenation of the corresponding oxime with hydrogen and a nickel catalyst in ethanolic ammonium acetate.

The corresponding β-isomer can be prepared by reduction of the same oxime but with sodium and amyl alcohol.

The mixture of α- and β-isomers can be prepared by reduction of the same oxime but with lithium aluminium hydride.

Pharmaceutically acceptable salts, N-oxides of the compounds of this invention may be formed conventionally.

The acid addition salts may be formed for example by reaction of the base compound of formula (I) with a pharmaceutically acceptable organic or inorganic acid.

Quarternary ammonium salts may be prepared by reaction of a compound of the present invention with the appropriate alkyl, aryl, aralkyl chloride, bromide or iodide. This reaction may be carried out in a solvent, such as acetone, methanol, ethanol, dimethylformamide at ambient or elevated temperature with or without pressure.

N-oxides of the nitrogen atom of the bicyclic ring system are produced by reaction of a compound of formula (I) with an organic peracid, such as m-chloroperbenzoic acid in, for example, a chlorinated hydrocarbon solvent at below ambient temperature.

The compounds of the present invention are dopamine antagonists and depending on their balance between peripheral and central action, may be used in the treatment of disorders relating to impaired gastro-intestinal motility, such as retarded gastric emptying, dyspepsia, flatulence, oesophagal reflux and peptic ulcer and emesis, and/or in the treatment of disorders of the central nervous system, such as psychosis.

Those compounds of the present invention which are of particular interest for their CNS activity, are those of formula (II).

Those compounds of the present invention which are of particular interest for their beneficial effect on gastric motility are the quaternary ammonium salts and N-oxides of such compounds.

The invention also provides a pharmaceutical composition comprising a compound of the present invention, or a pharmaceutically acceptable salt or N-oxide thereof or a solvate of any of the foregoing and a pharmaceutically acceptable carrier.

Such compositions are prepared by admixture and are suitably adapted for oral, rectal or parenteral administration, and as such may be in the form of tablets, capsules, oral liquid prparations, powders, granules, lozenges, reconstitutable powders, injectable and infusable solutions or suspensions or suppositories. Orally administerable compositions are preferred.

Tablets and capsules for oral administration are usually presented in a unit dose, and contain conventional excipients such as binding agents, fillers, tabletting agents, lubricants, disintegrants, and wetting agents. The tablets may be coated according to well known methods in the art. Oral liquid preparations are usually in the form of aqueous or oily suspensions, solutions, emulsions, syrups, or elixirs, or are presented as a dry product for reconstitution with water or other suitable vehicle before use. Such liquid preparations may contain conventional additives such as suspending agents, emulsifying agents, non-aqueous vehicles (which may include edible oils), preservatives, and flavouring or colouring agents.

A favoured form of the composition of this invention is a hard gelatin capsule containing the active agent. The active agent may be in the form of a powder, granulate or the like and may advantageously be in intimate mixture with a lubricant such as magnesium stearate.

A further favoured form of the composition of this invention is a tablet containing the active agent. The active agent may be in the form of a recompressed granulate of the active ingredient in intimate mixture

with a lubricant such as magnesium stearate, a filler such as microcrystalline cellulose and a disintegrant such as sodium starch glycollate.

For parenteral administration, fluid unit dose forms are prepared containing a compound of the present invention and a sterile verticle. The compound, depending on the vehicle and the concentration, can be either suspended or dissolved. Parenteral solutions are normally prepared by dissolving the compound in the vehicle and filter sterilizing before filling into a suitable vial or ampoule and sealing. Advantageously, adjuvants such as a local anaesthetic, preservatives and buffering agents are also dissolved in the vehicle.

Parenteral suspensions are prepared in substantially the same manner except that the compound is suspended in the vehicle instead of being dissolved and sterilized by exposure to ethylene oxide before suspending in the sterile vehicle. Advantageously, a surfactant or wetting agent is included in the composition to facilitate uniform distribution of the compound of the invention.

The invention further provides a method of treatment or prophylaxis of emesis or disorders of the central nervous system in mammals, such as humans, which comprises the administration of an effective amount of a compound of the present invention, or a pharmaceutically acceptable acid addition salt thereof, or a solvate of either of the foregoing.

The invention further provides a method of treatment or prophylaxis of disorders related to impaired gastro-intestinal motility in mammals, such as humans, which comprises the administration of an effective amount of a compound of the present invention, or a pharmaceutically acceptable salt thereof, or N-oxide thereof, or a solvate of any of the foregoing.

An amount effective to treat the disorders hereinbefore described depends on the relative efficacies of the compounds of the invention, the nature and severity of the disorder being treated and the weight of the mammal. However, a unit dose will normally contain 0.1 to 20 mg for example 0.5 to 10 mg, of the compound of the invention. Unit doses will normally be administered more than once a day, for example 2, 3, 4, 5 or 6 times a day such that the total daily dose is normally in the range 0.01 to 10 mg/kg per day.

The invention provides a compound of the present invention or a pharmaceutically acceptable salt thereof, or a solvate of either of the foregoing for the treatment or prophylaxis of emesis or disorders related to impaired gastrointestinal motility or of the central nervous system.

The compound of the present invention also have the ability to potentiate the effect of conventional analgesics in migraine treatment when administered concurrently with the analgesic.

Accordingly, the present invention also provides a pharmaceutical composition comprising a compound of the present invention or a pharmaceutically acceptable salt or N-oxide thereof or a solvate of any of the foregoing, and an analgesic.

The compound of the present invention and the analgesic such as aspirin or paracetamol, are present in the composition in amounts generally similar to their usual effective dose.

The composition can be a combination product, for example a tablet or capsule containing both a compound of the invention and an analgesic for oral administration, or a twin pack comprising the two active ingredients made up for separate administration.

The invention accordingly provides a method of treatment of migraine in mammals including humans comprising the administration of an effective amount of a compound of the invention or a pharmaceutically acceptable salt or N-oxide thereof or a solvate of any of the foregoing and an analgesic.

The following examples illustrate the preparation of compounds of the invention and the following descriptions illustrate the preparation of intermedaites.

The following process examples illustrate analogous processes for the preparation of intermediates to the compounds of the invention.

All potentially chiral compounds in the description and examples are produced and reacted as racemates.

Process Example 1

2,5-Dimethoxy-2.5-dihydro-3-methylfuran (P1)

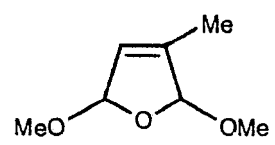

(P1)

A solution of 3-methylfuran (28.5 g) in ether (60 ml) and dry methanol (75 ml) at −40°C was treated dropwise with a cold solution of bromine (48 g) in methanol (195 g) over 45 minutes, keeping the temperature below −25°C. After 20 minutes a slow stream of ammonia was passed into the stirred solution keeping the temperature below −25°C, until the pH reached *ca* 9. Ether (200 ml) was added and the mixture was filtered and the filtrate evaporated to dryness *in vacuo* and distilled to afford the title compound as a colourless oil (41 g), (B.p. 60—68°C, 12 mm). δ($CDCl_3$) 1.8 (3H, s), 3.35 (6H, s), and 5.35—5.8 (3H, m).

Process Example 2

2,5-Dimethoxy-3-methyl tetrahydrofuran (P2)

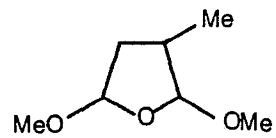

(P2)

A solution of 2,5-dimethoxy-2,5-dihydro-3-methylfuran (P1) (35 g) in methanol (250 ml) was hydrogenated over 10% palladium-on-carbon (5 g) at atmospheric pressure and temperature. When uptake of hydrogen (8 litres) had ceased (3 hours) the mixture was filtered (celite) and evaporated to dryness *in vacuo* to afford the title compound (35 g) which was used immediately in the next reaction. δ($CDCl_3$) 0.9 (3H, d, J=6Hz), 1.4—2.5 (*ca* 3H, m), 3.4 (6H, brs), and 3.9—5.2 (*ca* 2H, m).

Process Example 3

6-exo-methyl-8-(4′-methylbenzyl)-8-aza-bicyclo-[3.2.1]-octan-3-one (P3)

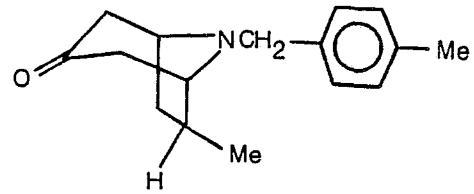

(P3)

A solution of 4-methylbenzylamine (28.4 g) in dilute hydrochloric acid (5*N*, 47 ml) was added to a solution of 2,5-dimethoxy-3-methyl tetrahydrofuran (P2) (33 g) in water (100 ml) containing 2 drops *conc.* hydrochloric acid, with stirring. A solution of 1,3-acetonedicarboxylic acid (34 g) and sodium acetate (19.2 g) in water (100 ml) was added and the volume of the solution was made up to 1.25 litres with water. It was stirred for 24 hr at room temperature. A further quantity of dilute hydrochloric acid (3 ml) was added and the mixture stirred for a further 24 hr. It was made acidic and washed with ether. The acidic solution was basified and extracted with ether. The ether extract was washed with water, dried ($Na_2SO_4$), evaporated to dryness *in vacuo*, and chromatographed on silica gel (300 g) in ethyl acetate — pentane (1:9). The early fractions were combined and re-crystallised from ether — pentane to afford the title compound (7.0 g), m.p. 71—73°C.

Found: C, 79.5; H, 9.15; N, 5.94. $C_{16}H_{21}NO$ requires C, 78.95; H, 8.7; N, 5.75%, νmax. 1710 $cm^{-1}$; δ(250 mHz) ($CDCl_3$) 1.1 (3H, d, *J*=5Hz), 1.6 (1H, m), 1.85 (1H, m), 1.9 (1H, m), 2.13 (2H, br.t, J=14Hz), 2.35 (3H, s), 2.7 (2H, d.d. J=14, 4Hz), 3.09 (1H, br.s, $W_{\frac{1}{2}}$=*ca* 6Hz), 3.58 (1H, br.t, $W_{\frac{1}{2}}$=*ca* 12Hz), 3.92 (2H, q), 7.15 1H, d, *J*=7Hz) and 7.32 (1H, d, J=7Hz).

Process Example 4

6-exo-Methyl-8-(4′-methylbenzyl)-8-aza-bicyclo-[3.2.1]-octan-3-one oxime (P4)

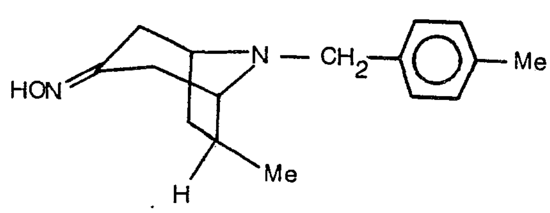

(P4)

A solution of 6-*exo*-methyl-8-(4′-methylbenzyl)-8-aza-bicyclo-[3.2.1]-octan-3-one (P3) (3.0 g), hydroxylamine hydrochloride (0.9 g), and pyridine (0.5 ml) in ethanol (75 ml) was heated at reflux for 1 hour. The solution was evaporated to dryness *in vacuo* and shaken with ethyl acetate — sodium carbonate solution. The organic phase was washed with water, dried ($Na_2SO_4$) and evaporated to dryness *in vacuo*. Recrystallisation from ethyl acetate — pentane gave the *oxime* (3.05 g), m.p. 107—114°C.

Found: C, 74.6; H, 8.6; N, 10.85. $C_{16}H_{22}N_2O$ requires C, 74.4; H, 8.6; N, 10.85%.

Process Example 5

3-β-Amino-6-exo-methyl-8-(4'-methylbenzyl)-8-azabicyclo-[3.2.1]-octane (P5)

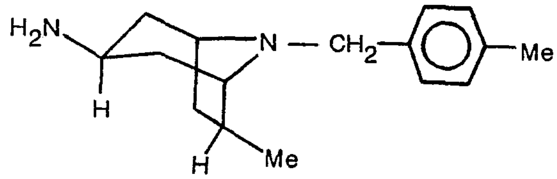

(P5)

A solution of 6-*exo*-methyl-8-methyl-(4'-methylbenzyl)-8-azabicyclo-[3.2.1]-octan-3-one oxime (P4) (2.9 g) in the refluxing amyl alcohol (100 ml) under nitrogen was treated with sodium (2.9 g) portionwise over 1.5 hour. The solution was cooled and evaporated to dryness *in vacuo*.

The residue was dissolved in dilute hydrochloric acid, washed with ether, basified with excess sodium carbonate, and extracted with methylene chloride. The organic fraction was washed with water, dried ($Na_2SO_4$) and evaporated to dryness *in vacuo* to afford crude title compound (2.5 g), which was used as required without purification.

Description 1

7-exo-Methyl-9-(4'-methylbenzyl)-9-aza-bicyclo-[3.3.1]-nonan-3-one (D1)

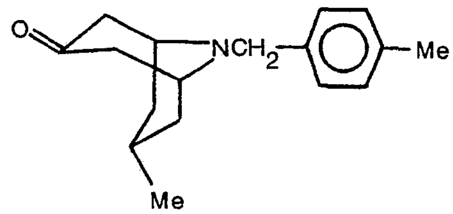

(D1)

A mixture of 2-ethoxy-4-methyl-2,3-dihydropyran (35 g) in water (70 ml) containing conc. hydrochloric acid (3 ml) was stirred at *ca* 40°C for 2.5 hours. A solution of 4-methylbenzylamine (28.4 g) in dilute hydrochloric acid (5*N*, 47 ml) was added followed by a solution of 1,3-acetonedicarboxylic acid (34 g) and sodium acetate (19.2 g) in water (100 ml) and the volume of solution was made up to 1.25 litres with water. The solution was stirred at room temperature for 48 hours, acidified with dilute hydrochloric acid, and washed with ether. The acidic solution was basified, extracted with methylene chloride, chromatographed and recrystallised using the procedure of Process Example 3 to afford the title compound (28 g), m.p. 79—81°C.

(Found: C, 79.45; H, 9.0; N, 5.45. $C_{17}H_{23}NO$ requires C, 79.35; H, 9.0; N, 5.45%), δ ($CDCl_2$—250MHz) 0.86 (3H, d, *J*6Hz); 1.45—1.75 (5H, m); 2.25 (2H, d, *J*=18Hz), 2.36 (3H, S), 2.72 (2H, d.d. J=18,8Hz), 3.33 (2H, br.s, $W\frac{1}{2}$=14Hz), 3.86 (2H, s), 7.14 (2H, d, *J*=8Hz and 7.28 (2H, d, J=8Hz).

Description 2

7-exo-Methyl-9-(4'-Methylbenzyl)-9-aza-bicyclo-[3.3.1]-nonan-3-one oxime (D2)

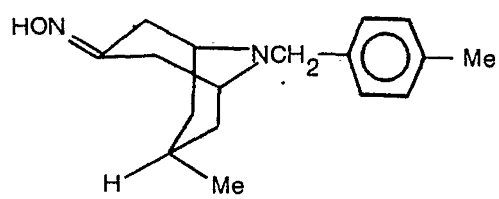

(D2)

This compound was prepared using the procedure of Process Example 4. Recrystallisation from ethyl acetate-pentane gave the title compound, m.p. 102—103°C.

Description 3

3-β-Amino-7-exo-Methyl-9-(4'-Methylbenzyl)-9-aza-bicyclo-[3.3.1[-nonane (D3)

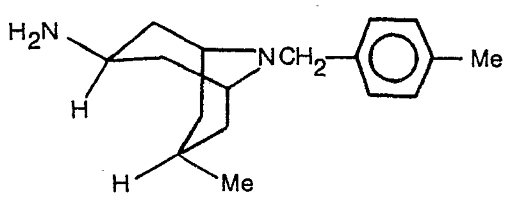

(D3)

This compound was prepared using the procedure of Process Example 5. The product had m.p. 36—39°C.

Description 4

(6-Endo) and (6-Exo)-Methyl-9-(4′-Methylbenzyl)-9-aza-bicyclo-[3.3.1]-nonan-3-one (D4)

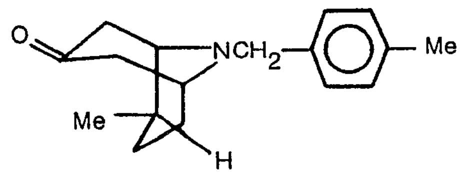

*endo* (D4a)

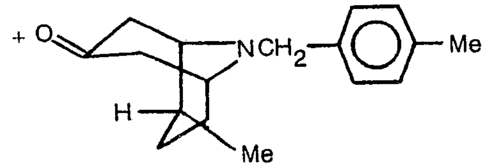

*exo* (D4b)

Reaction between 2-ethoxy-5-methyl-2,3-dihydropyran (23 g), 4-methylbenzylamine (18.7 g) and 1,3-acetonedicarboxylic acid (22.3 g) in the manner of Description 1 gave a mixture (17.45 g) of 6-*endo* and 6-*exo* isomers which were chromatographed on silica gel in ethyl acetate-pentane (1:4). The 6-*exo*-compound (D4b) (1.4 g) was eluted first, m.p. 83—88°C (from pentane). (Found: C, 79.55; H, 9.15; N, 5.3. $C_{17}H_{23}NO$ requires C, 79,35; H, 9.0; N, 5.45%), δ ($CDCl_3$; 250mHz) 0.85 (3H, d, *J*=8Hz) 1.1 (1H, m), 1.52 (2H, m), 2.0 (2H, m), 2.2 (1H, d, *J*=17Hz), 2.34 (3H, s), 2.38 (1H, d, J=17Hz); 2.52 (1H, d, d, J=17,17Hz); 2.7 (1H, dd, J=17,7Hz); 3.05 (1H, br.s. $W\frac{1}{2}$=12Hz), 3.3 (1H, br.s. $W\frac{1}{2}$=13Hz), 3.9 (2H, s), 7.15 (2H, d, *J*=8Hz), and 7.29 (2H, d, *J*=8Hz).

Further elution gave the 6-*endo*-compound (D4a) (16 g) m.p 66—67° (from ether-pentane), (Found: C, 79.4; H, 9.45; N, 5.45. $C_{17}H_{23}NO$ requires C, 79.35; H, 9.0; N, 5.45), δ ($CDCl_3$; 250mHz) 1.17 (3H, d, *J*=7Hz); 1.25 (1H, m), 1.42 (1H, m), 1.42 (1H, m), 1.65 (1H, m), 1.77 (1H, m), 2.06 (1H, m), 2.16 (2H, d, *J*=17Hz), 2.35 (3H, s), 2.75 (2H, m), 3.02 (1H, br.d, *J*=7Hz, $W\frac{1}{2}$=11Hz), 3.33 (1H, br.s. $W\frac{1}{2}$=14Hz), 3.77 (1H, d, *J*=13Hz), 3.85 (1H, d, J=13Hz), 7.15 (2H, d, *J*=8Hz) and 7.3 (2H, dl J=8Hz).

Description 5

(6-Endo) and (6-Exo)-Methyl-9-(4′-Methylbenzyl)-9-aza-bicyclo-[3.3.1]-nonan-3-one oxime (D5)

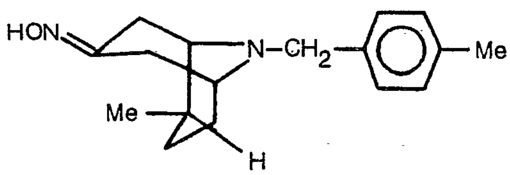

(D5a)

*endo*

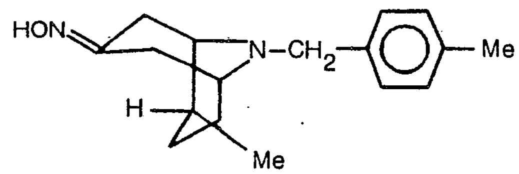

(D5b)

*exo*

These compounds were prepared using the procedure of Process Example 4. Recrystallisation from ethyl acetate pentane gave the 6-*endo* methyl isomer oxime (D5a), m.p. 109—111°C, and 6-*exo*-methyl isomer oxime (D5b), m.p. 120—122°C.

Description 6

3-β-Amino-(6-endo) and (6-exo)-methyl-9-(4′-methylbenzyl)-9-aza-bicyclo-[3.3.1]-nonane (D6a) and (D6b)

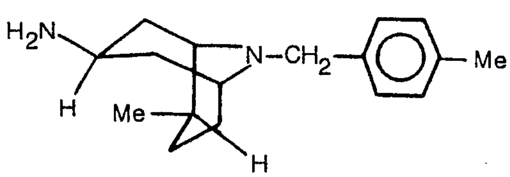

(D6a)

*endo*

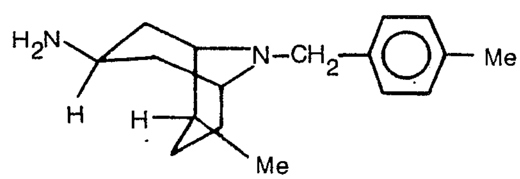

(D6b)

*exo*

Both compounds were prepared for the corresponding oximes, using the procedure of Process Example 5 and were oils.

Example 1

4-Amino-5-Chloro-2-Methoxy-N-[7′-Methyl-9′-(4-Methylbenzyl)-9′-aza-bicyclo-[3.3.1]-non-3-β-yl]-benzamide (E1)

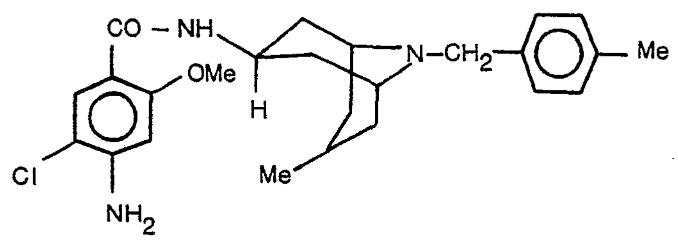

(E1)

A suspension of 4-amino-5-chloro-2-methoxy benzoic acid (1.14 g) in methylene chloride (25 ml) and triethylamine (2 ml) at 0°C was treated dropwise with ethyl chloroformate (0.61 g) and the resulting solution was allowed to reach room temperature over 1 hour.

A solution of 3-β-amino-7-methyl-9-(4′-methylbenzyl)-9-aza-bicyclo-[3.3.1]-nonane (1.4 g) in methylene chloride (5 ml) was added to the stirred solution, dropwise, over 5 min. After a further 0.5 hour, the mixture was treated with methylene chloride (200 ml) and washed with 10% sodium hydroxide, water, dried and evaporated to dryness *in vacuo*. The product was recrystallised from ethyl acetate-pentane to give the title compound (2.01 g), m.p. 224—226°C.

The methyl group at position −7′ is believed to be in the *exo*-configuration.

(Found: C 67.95; H, 7.45; N, 9.5; Cl, 8.0. $C_{25}H_{32}N_3O_2Cl$ requires C, 67.95; H, 7.3; N, 9.5; Cl 8.0%).

Example 2

4-Amino-5-Chloro-2-Methoxy-N-[6′-endomethyl-9′-(4-methylbenzyl)-9′-aza-bicyclo-[3.3.1]-non-3-β-yl]benzamide (E2)

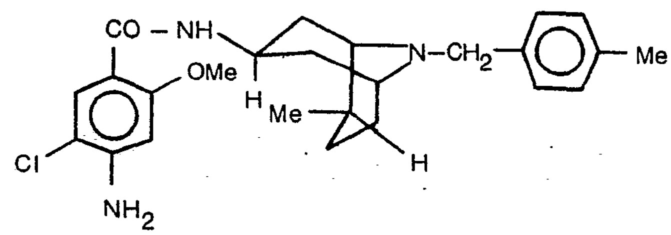

(E2)

This compound was prepared from the amine (D6a) following the procedure of Example 1; m.p. 199—200°C, (Found: C, 68.0; H, 7.3; N, 9.5; Cl, 8.1. $C_{25}H_{23}N_2O_2Cl$ requires C, 67.95; H, 7.3; N, 9.5; Cl 8.0%).

Example 3

4-Amino-5-Chloro-2-Methoxy-N-[6′-exo-methyl-9′-(4-methylbenzyl)-9′-aza-bicyclo-[3.3.1]-non-3-β-yl]benzamide (E3)

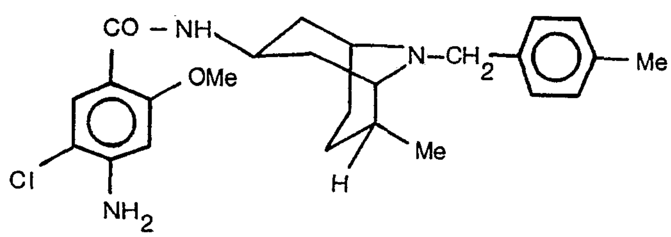

(E3)

This compound was prepared from the amine (D6b) following the procedure of Example 1; m.p. 185—186°C, (Found: C, 67.8; H, 7.3; N, 9.4; Cl. 7.85. $C_{25}H_{32}N_3O_2Cl$ requires C, 67.95; H, 7.3; N, 9.5; Cl, 8.0%).

PHARMACOLOGICAL DATA

The results in the following table are an illustration of the anti-psychotic activity of the present compounds as shown by Inhibition of Apormorphine Induced Climbing in the Mouse, a standard test.

Inhibition of apomorphine induced climbing in the mouse

The test is based on that described by Protais, P., Costefin, J. and Schwartz, J. C. (1976), Psychopharmacology, *50*, 1—6.

When mice are given a dose of 1 mg/kg apomorphine and then placed in an enclosed environment, such as an inverted wire cage, they are seen to climb around the walls. This behavioural phenomenon is

thought to be a consequence of the stimulation of post-synaptic Dopamine (D.A.) receptors in the nucleus accumbens. Inhibition of apomorphine induced climbing is therefore indicative of post-synaptic D.A. receptor blockade in the accumbens.

Groups of 10 male CD1 mice, weighing 25—30 g were pre-treated orally with either graded doses of the test compound or vehicle, at appropriate time intervals before the subcutaneous administration of a sub-maximal dose of apomorphine (1 mg/kg). Immediately after the apomorphine injection the mice were placed in wire 'climbing cages' and each animal was scored for climbing behaviour at 10 and 20 minutes post apomorphine as follows:—

Four paws on cage floor = 0
Fore paws on cage wall = 1
Four paws on cage wall = 2

The total score was calculated for each group of mice and expressed as a percentage inhibition of climbing.

$$\% \text{ inhibition} = 100 - \frac{\text{Total score for test compound}}{\text{Total score for apomorphine control}} \times 100$$

ED50's and fiducial limits were calculated according to the method of Litchfield and Wilcoxon, the ED50 being the dose that produced a 50% inhibition of apomorphine-induced climbing.

The table shows the dose for 50% inhibition at 0.5 and 4.0 hours post dosing

| Compound of Example No. | $ED_{50}$ mg/kg p.o. $\frac{1}{2}$ hr |
|---|---|
| 1 | 0.6 p.o. $\frac{1}{2}$ hr |
| 2 | 1.1 p.o. $\frac{1}{2}$ hr |
| 3 | 2.75 p.o. $\frac{1}{2}$ hr |

Toxicity

No toxic effects were observed in the test reported above.

**Claims**

1. A compound of formula (I), or a pharmaceutically acceptable salt thereof, or a solvent adduct or N-oxide thereof, or a solvate of any of the foregoing:

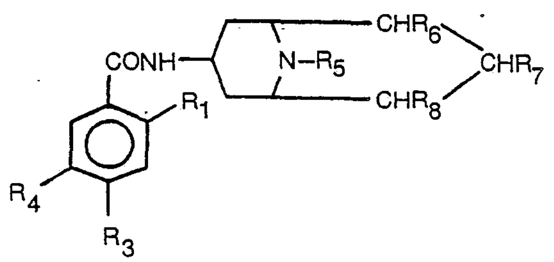

(I)

wherein

$R_1$ is $C_{1-6}$ alkoxy;
$R_3$ is carboxylic $C_{1-7}$ acylamino or amino;
$R_4$ is halogen or hydrogen;
$R_5$ is $—CH_2R_{11}$, $R_{11}$ being phenyl substituted by one substituent selected from halogen and $C_{1-4}$ alkyl; and

one of $R_6$, $R_7$ and $R_8$ is $C_{1-4}$ alkyl and the other two are the same or different and are hydrogen or $C_{1-4}$ alkyl.

2. A compound according to claim 1, of formula (II), a pharmaceutically acceptable acid addition salt thereof or a solvate of either of the foregoing:

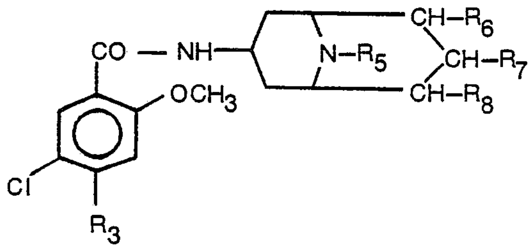

(II)

wherein $R_3$ is amino or $C_{1-4}$ alkanoylamino, and $R_5$ to $R_8$ are as defined in claim 1.

3. A compound according to claim 2 wherein $R_5$ is selected from p-fluorobenzyl, p-chlorobenzyl and p-methylbenzyl.

4. A compound according to claim 3, which is 4-Amino-5-chloro-2-methoxy-N-{7'-methyl-9'-(4-methylbenzyl)-9'-azabicyclo-{3.3.1}-non-3'-β-yl}benzamide, or a pharmaceutically acceptable acid addition salt thereof, or a solvate of any of the foregoing.

5. A compound according to any one of claims 1 to 4, wherein $R_6$, $R_7$ or $R_8$ when other than hydrogen is in the exo configuration.

6. A process for the preparation of a compound according to claim 1, or a pharmaceutically acceptable salt or N-oxide thereof or a solvate of any of the foregoing, which comprises reacting a compound of formula (V):

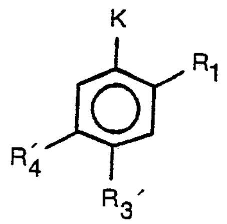

(V)

with a compound of formula (VI):

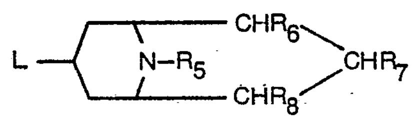

(VI)

wherein $R_1$ and $R_5$ to $R_8$ are as defined;

K is $COQ_1$ in which $Q_1$ is a leaving group, and L is $NH_2$;

$R_3'$ and $R_4'$ are the same or different and are selected from hydrogen, halogen, carboxylic $C_{1-7}$ acylamino, nitro, hydroxy or amino; converting $R_3'$ and/or $R_4'$ as necessary to $R_3$ and $R_4$ respectively; optionally converting $R_3$ or $R_4$ in the thus formed compound to another $R_3$ or $R_4$ within formula (I); and optionally forming a pharmaceutically acceptable salt or N-oxide of the thus formed compound.

7. A pharmaceutical composition comprising a compound according to claim 1, or a pharmaceutically acceptable salt or N-oxide thereof or a solvate of any of the foregoing and a pharmaceutically acceptable carrier.

8. A compound according to claim 1 or a pharmaceutically acceptable acid addition salt thereof, or a solvate of either of the foregoing for the treatment or prophylaxis of emesis or disorders related to impaired gastro-intestinal motility or of the central nervous system.

9. A compound according to claim 1 or a pharmaceutically acceptable salt thereof, or a solvent adduct or N-oxide thereof, or a solvate of any of the foregoing, for use as an active therapeutic substance.

**Patentansprüche**

1. Eine Verbindung der Formel (I) oder deren pharmazeutisch verträgliches Salz oder ein auflösendes Addukt oder deren N-Oxid oder ein Solvat irgendeiner der vorstehenden:

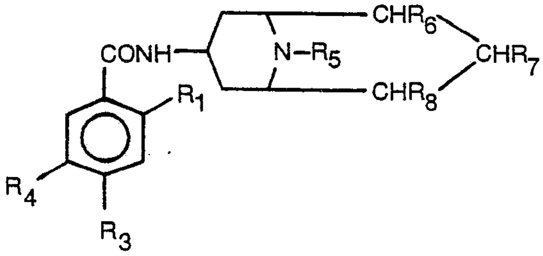

(I)

in der

$R_1$ $C_{1-6}$-Alkoxy ist;

$R_3$ carboxylisches $C_{1-7}$-Acylamino oder Amino ist;

$R_4$ Halogen oder Wasserstoff ist;

$R_5$ —$CH_2R_{11}$ ist, wobei $R_{11}$ Phenyl ist, das durch einen Substituenten ausgewählt aus Halogen und $C_{1-4}$-Alkyl substituiert ist; und

einer von $R_6$, $R_7$ und $R_8$ $C_{1-4}$-Alkyl ist und die beiden anderen gleich oder verschieden sind und Wasserstoff oder $C_{1-4}$-Alkyl sind.

2. Eine Verbindung nach Anspruch 1, der Formel (II), deren pharmazeutisch verträgliches Säureadditionssalz oder ein Solvat der beiden vorstehenden

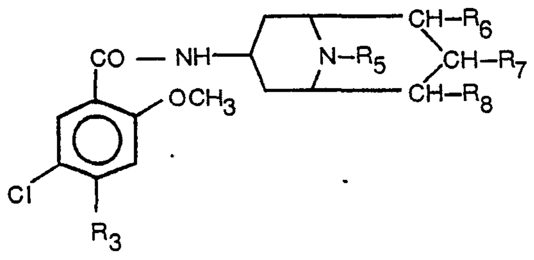

(II)

in der $R_3$ Amino oder $C_{1-4}$-Alkanoylamino ist und $R_5$ bis $R_8$ wie in Anspruch 1 definiert sind.

3. Eine Verbindung nach Anspruch 2, wobei $R_5$ aus p-Fluorbenzyl, p-Chlorbenzyl und p-Methylbenzyl ausgewählt ist.

4. Eine Verbindung nach Anspruch 3, die 4-Amino-5-chlor-2-methoxy-N-{7'-methyl-9'-(4-methylbenzyl)-9'-azabicyclo[3.3.1]-non-3'-β-yl}-benzamid oder dessen pharmazeutisch verträgliches Säureadditionssalz oder ein Solvat irgendeines der vorstehenden ist.

5. Eine Verbindung nach irgendeinem der Ansprüche 1 bis 4, in der $R_6$, $R_7$ oder $R_8$, wenn sie anders als Wasserstoff sind, in der exo-Konfiguration sind.

6. Ein Verfahren zur Herstellung einer Verbindung nach Anspruch 1 oder deren pharmazeutisch verträgliches Salz oder N-Oxid oder ein Solvat von irgendeiner der vorstehenden, das umfaßt das Umsetzen einer Verbindung der Formel (V):

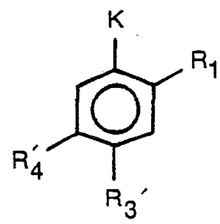

(V)

mit einer Verbindung der Formel (VI)

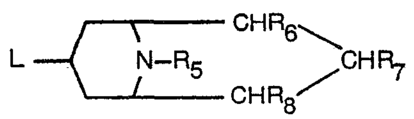

(VI)

in der $R_1$ und $R_5$ bis $R_8$ wie definiert sind;

K $COQ_1$ ist, in der $Q_1$ eine nukleophile Abgangsgruppe ist und

L $NH_2$ ist;

$R_3'$ und $R_4'$ gleich oder verschieden sind und ausgewählt aus Wasserstoff, Halogen, carboxylischem $C_{1-7}$-Acylamino, Nitro, Hydroxy oder Amino sind;

das Umwandeln von $R_3'$ und/oder $R_4'$ — wenn erfordereich — in $R_3$ bzw. $R_4$; gegebenenfalls Umwandeln von $R_3$ oder $R_4$ bei der derart gebildeten Verbindung in ein anderes $R_3$ oder $R_4$ innerhalb der Formel (I); und gegebenenfalls Bilden eines pharmazeutisch verträglichen Salzes oder N-Oxids der derart gebildeten Verbindung.

7. Eine pharmazeutische Zusammensetzung, umfassend eine Verbindung nach Anspruch 1 oder deren pharmazeutisch verträgliches Salz oder N-Oxid oder ein Solvat irgendeiner der vorstehenden und einen pharmazeutisch verträglichen Träger.

8. Eine Verbindung nach Anspruch 1 oder deren pharmazeutisch verträgliches Säureadditionssalz oder ein Solvat der beiden vorstehenden zur Behandlung oder Vorbeugung bei Erbrechen oder Störungen, bezogen auf eine gestörte gastrointestinale Beweglichkeit, oder des Zentralnervensystems.

9. Eine Verbindung nach Anspruch 1 oder deren pharmazeutisch verträgliches Salz oder deren auflösendes Addukt oder N-Oxid oder ein Solvat irgendeiner der vorstehenden zur Verwendung als eine aktive therapeutische Substanz.

**Revendications**

1. Composé de formule (I), ou un sel acceptable du point de vue pharmaceutique de celui-ci, ou un adduct de solvant ou un N-oxyde de celui-ci ou un solvat de l'un quelconque des précédents:

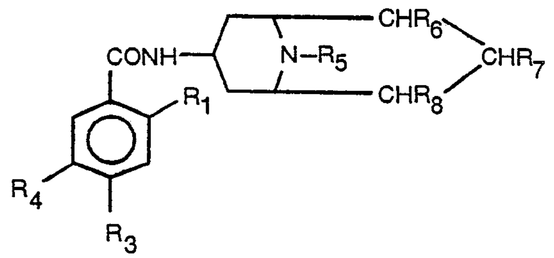

(I)

dans laquelle

$R_1$ est un groupe alcoxy en $C_{1-6}$;

$R_3$ est un groupe acylamino en $C_{1-7}$ carboxylique ou amino;

$R_4$ est un atome d'halogène ou d'hydrogène;

$R_5$ est un groupe $—CH_2R_{11}$, $R_{11}$ étant un groupe phényle substitué par un substituant choisi parmi un atome d'halogène et un groupe alcoyle en $C_{1-4}$; et

l'un des $R_6$, $R_7$ et $R_8$ est un groupe alcoyle en $C_{1-4}$ et les deux autres sont identiques ou différents et ils sont un atome d'hydrogène ou un groupe alcoyle en $C_{1-4}$.

2. Composé suivant la revendication 1, de formule (II), un sel d'addition acide acceptable du point de vue pharmaceutique de celui-ci ou un solvat de l'un quelconque des précédents:

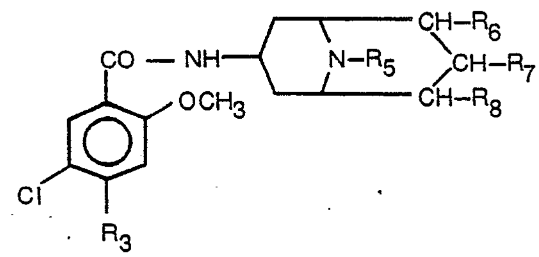

(II)

Dans laquelle $R_3$ est un groupe amino ou alcanoylamino en $C_{1-4}$, et $R_5$ à $R_8$ sont tels qu'ils sont définis dans la revendication 1.

3. Composé suivant la revendication 2, dans lequel $R_5$ est choisi parmi les groupes p-fluorobenzyle, p-chlorobenzyle et p-méthylbenzyle.

4. Composé suivant la revendication 3, qui est le 4-amino-5-chloro-2-méthoxy-N-{7'-méthyl-9'-(4-méthylbenzyl)-9'-azabicyclo{3.3.1}-non-3'-β-yl}benzamide, ou un sel d'addition acide acceptable du point de vue pharmaceutique de celui-ci, ou un solvat de l'un quelconque des précédents.

5. Composé suivant l'une quelconque des revendications 1 à 4, dans lequel $R_6$, $R_7$ ou $R_8$ est en configuration exo, lorsqu'il est autre qu'un atome d'hydrogène.

6. Procédé pour la préparation d'un composé suivant la revendication 1, ou d'un sel ou d'un N-oxyde acceptable du point de vue pharmaceutique de celui-ci ou d'un solvat de l'un quelconque des précédents, qui comprend la réaction d'un composé de formule (V)

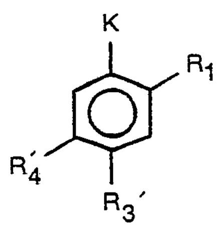

(V)

avec un composé de formule (VI)

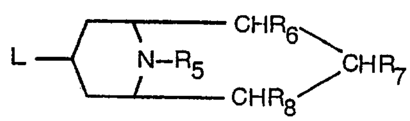

(VI)

dans laquelle $R_1$ et $R_5$ à $R_8$ sont tels que définis:

K est $COQ_1$, où $Q_1$ est un groupe mobile et L est $NH_2$ $R_3'$ et $R_4'$ sont identiques ou différents et sont choisis parmi un atome d'hydrogène, d'halogéne, un groupe acylamino en $C_{1-7}$ carboxylique, nitro, hydroxy ou amino; la conversion de $R_3'$ et/ou $R_4'$, si cela est nécessaire, en $R_3$ et $R_4$ respectivement; la conversion éventuelle de $R_3$ ou $R_4$ dans le composé ainsi formé en un autre $R_3$ ou $R_4$ dans la formule (I); et la formation éventuelle d'un sel ou d'un N-oxyde acceptable du point de vue pharmaceutique du composé anisi formé.

7. Composition pharmaceutique comprenant un composé suivant la revendication 1, ou un sel ou un N-oxyde acceptable du point de vue pharmaceutique ou un solvat de l'un quelconque des précédents et un support acceptable du point de vue pharmaceutique.

8. Composé suivant la revendication 1 ou un sel d'addition acide acceptable du point de vue pharmaceutique de celui-ci, ou un solvat de l'un quelconque des précédents, pour le traitement ou la prophylaxie des vomissements ou de troubles associés à une mobilité gastro-intestinale altérée ou troubles du système nerveux central.

9. Composé suivant la revendication 1 ou un sel acceptable du point de vue pharmaceutique de celui-ci, ou un adduct de solvant ou un N-oxyde de celui-ci, ou un solvat de l'un quelconque des précédents, utile comme substance thérapeutique active.